# EUROPEAN PATENT SPECIFICATION

(11) **EP 1 441 686 B1**
(45) Date of publication and mention of the grant of the patent: **02.07.2008**
(21) Application number: 02803154.0
(22) Date of filing: 11.10.2002
(51) Int. Cl.: A61Q 19/08, A61K 8/97

(54) **TOPICAL COSMETIC COMPOSITION WITH SKIN REJUVENATION BENEFITS**
TOPISCHE KOSMETISCHE ZUSAMMENSETZUNG MIT POSITIVEN HAUTVERJÜNGUNGSEIGENSCHAFTEN
COMPOSITION COSMETIQUE TOPIQUE PERMETTANT LE RAJEUNISSEMENT DE LA PEAU

(30) Priority: 09.11.2001 US 39746
(43) Date of publication of application: 04.08.2004
(73) Proprietor: AVON PRODUCTS, INC., New York, NY 10020-1196 (US)
(72) Inventor: LU, Michelle, Nanuet, New York 10954 (US); DUGGAN, Michelle, Middletown, NY 10940 (US); MENON, Gopinathan, K., Wayne, NJ 07470 (US); THEOPHILUS, Eugenia, H., Clemmons, NC 27012 (US); DOKKA, Sujatha, San Marcos, CA 92069 (US); WANG, Helen, Suffern, NY 10901 (US)
(74) Representative: Dr. Weitzel & Partner
(86) International application number: PCT/US2002/032579
(87) International publication number: WO 2003/041636

(56) References cited:
- DE-A- 10 021 560
- US-A- 4 942 153
- US-A- 5 547 997
- US-A- 5 891 440
- US-A- 5 925 348
- US-B1- 6 224 850
- DATABASE WPI Section Ch, Week 200145 Derwent Publications Ltd., London, GB; Class B04, AN 2001-421242 XP002310056 & JP 2001 131053 A (null) 15 May 2001 (2001-05-15)
- PATENT ABSTRACTS OF JAPAN vol. 0111, no. 33 (C-418), 25 April 1987 (1987-04-25) & JP 61 271210 A (POLA CHEM IND INC), 1 December 1986 (1986-12-01)
- PATENT ABSTRACTS OF JAPAN vol. 1995, no. 08, 29 September 1995 (1995-09-29) & JP 7 138180 A (MIKIMOTO PHARMACEUT CO LTD; others: 01), 30 May 1995 (1995-05-30)
- DATABASE JPAB [Online] XP002960660 & JP 4 308 511 A 30 October 1992
- DATABASE JPAB [Online] XP002960658 & JP 2000 226321 A 15 August 2000
- DATABASE WPI Week 199924, Derwent Publications Ltd., London, GB; AN 1999-280706, XP002219078 & JP 10 330 281 A (NOEVIR KK) 15 December 1998
- DATABASE JPAB [Online] XP002960659 & JP 61 027 210 A 01 December 1986

## Description

### BACKGROUND OF THE INVENTION

### 1. Field of the Invention

The present invention relates to topical compositions for providing rejuvenation benefits to the skin. More particularly, the present invention relates to compositions providing anti-aging benefits to the skin.

### 2. Description of the Prior Art

There is active contemporary interest in the cosmetics industry to develop products that may be applied topically to the skin that provide anti-aging, hydrating, and/or skin texturizing benefits. Cosmetic products, which enhance the appearance of skin, are increasingly in demand. Consumers are interested in mitigating or delaying the signs of aged or photo-aged skin, such as fine lines, wrinkles, drying, and sagging skin. During the aging process, the complexion of the skin, i.e., the color and appearance of the skin, deteriorates slowly from aging and/or exposure to sunlight. Cosmetic surgery can be used as a treatment for aged skin. However, such treatment is costly and carries the risks normally associated with anesthesia and surgery. Alternatively, cosmetic products that provide anti-aging benefits are highly desirable, to both manufacturers and consumers.

The number of cosmetic products directed to help the skin of consumers look younger and less wrinkled is steadily increasing. Commonly, such products contain exfoliating acids as active ingredients. Such anti-aging active Ingredients include, for example, a-hydroxy acids (e.g., lactic, glycolic, citric), b-hydroxy acids (e.g., salicylic, 5-n-octanoylsalicylic acids) and retinolds (retinoic acids; retinol). It is known that these anti-aging active ingredients have a significant disadvantage In that they frequently are associated with consumer discomfort characterized by burning, smarting, itching or sensation of tightness after application. There remains a general need in the cosmetics industry for products that retard or counter aging effects on the skin, and more specifically for products that produce such effects without undesirable side effects.

The DE 10021560A and the JP404308511A refer to extracts from neem frees. Plant extracts have been used in cosmetic compositions. U.S. Patent No. 5,709,864 to Patrice et al. is directed to compositions one effect of which is anti-aging activity, This patent provides dermatological compositions containing extract of a plant of the genus *Tephrosia*, particularly an extract of the species *Tephrosia purpurea.*

U.S. Patent No. 5,624,673 to Frederic et al*,* is directed to a method for the treatment of skin for anti-aging and other effects using a plant extract. This patent provides a treatment of the epidermis using an extract of various parts of a plant from the genus *Prunelle*, particularly an extract of the species *Prunella vulgaris.*

U.S. Patent No. 5,093,109 to Mausner is directed to cosmetic compositions for retarding the effects of aging on the complexion of the skin. This patent provides compositions that have anti-aging agents obtained from plant extracts. These anti-aging agents are flavenoid compounds provided by extracts of butcher broom, buckwheat, passion flower and combinations thereof.

U.S. Patent No. 5,925,348 to Babcock et al*.* is directed to a method of treating skin with an anti-aging component. This patent provides anti-aging components obtained from extracts of the Sacred Lotus plant, the Yellow Lotus plant, Yellow Lotus seeds and wheat germ oil. Extracts of animal organs are also provided as anti-aging components.

U.S. Patent Nos. 5,891,440 and 6,060,063 are directed to oral and topical phytoestrogen compositions having pomegranate oil obtained from pressed seed or from an extract of the seed and juice. The topical compositions may be applied to the skin, including the face and the vagina. The topical compositions may be used to relieve symptoms in menopausal women, including vaginal dryness and lack of skin tone.

WO 00/164472 is directed to a method of treating dermatological conditions via ingestion of pomegranate. Treatable conditions include dry and yellowish skin, hyperpigmented skin and wrinkles.

Japanese Publication 143491 is directed to anti-aging cosmetics. The anti-aging inhibitor is essence of pomegranate seeds.

German Publications 4330597, 4312109 and 4204255 are directed to hair growth promoting compositions having a large number of plant constituents, including pomegranate juice, core, peel, pips, inner skin and paraffin-based oil.

Soviet Publication 1602533 is directed to face creams for greasy skin. The creams contain stearin, emulsion wax, triethanolamine, carbonated extracts of pomegranate skins and seed and of parsley.

In spite of the various anti-aging cosmetic products on the market for the treatment of skin, there remains a need for effective anti-aging compositions that can be applied topically to the skin. More particularly, there remains a need for topically applied cosmetic compositions that have anti-aging and skin texture benefits without the frequent irritation associated with the use of exfoliating acids. The present invention achieves these benefits by a topical composition comprising natural ingredients, preferably botanical ingredients, as active components, instead of such prior art acids discussed above. In other words, the present composition is substantially free of such acids.

### SUMMARY OF THE INVENTION

It is an object of the present invention to provide cosmetic compositions that improve the appearance of skin and remediate the effects of aging.

These and other objects and advantages of the present invention, and equivalents thereof, are achieved by anti-aging cosmetic compositions having botanical ingredients, and the use of such compositions for topical application to the skin.

The composition of the present invention has an effective amount of neem seed cell broth, and one or more botanical ingredients selected from the group consisting of grape seed extract, pomegranate fruit extract, cucumber extract, carrot extract, iris root extract, white birch extract, *Salvia miltorrhiza* extract and Laminaria algae extract. In a more preferred embodiment, the neem seed cell broth and the other botanical Ingredient(s) are combined with a cosmetically acceptable vehicle.

### DETAILED DESCRIPTION OF THE INVENTION

The present invention provides anti-aging benefits to and improves the aesthetic appearance of the skin, In particular, the present invention provides compositions and methods for treating skin to prevent, inhibit, reduce and/or ameliorate the signs of dermatological aging due to, for example, chronological aging, hormonal aging, and/or photoaging. Such signs of aging include, but are not limited to skin fragility; loss of collagen and/or elastin; skin atrophy; appearance and/or depth of lines and/or wrinkles, including fine lines; skin discoloration, including dark eye circles; skin sagging; skin fatigue and/or stress, e.g. skin breakout due to environmental stress, such as pollution and/or temperature changes; skin dryness; skin flakiness; cellular aging; loss of skin tone, elasticity and/or luster; loss of skin firmness; poor skin texture; loss of skin elasticity and/or resiliency; and thin skin.

The benefits and improvements to the aesthetic appearance of skin can be manifested In any of the following; reduction in pore size; improvement In skin tone, radiance, clarity and/or tautness; promotion of anti-oxidant activity; improvement In skin firmness, plumpness, suppleness, and/or softness; improvement in procollagen and/or collagen production; improvement in skin texture and/or promotion of retexturization; improvement in skin barrier repair and/or function; improvement in appearance of skin contours; restoration of skin luster and/or brightness: replenishment of essential nutrients and/or constituents in the skin decreased by aging and/or menopause; increase in cell proliferation and/or multiplication: improvement in skin moisturization; promotion and/or acceleration of cell turnover; enhancement of skin thickness; increase in skin elasticity and/or resiliency; and enhancement of exfoliation, with or without the use of alpha or beta hydroxy acids, keto acids or other exfoliants.

The present invention also includes methods of treating skin with the topical application of the present cosmetic compositions.

Neem seed cell broth is combined with one or more additional botanical ingredients selected from one or more of the following: grape seed extract, pomegranate fruit extract, cucumber extract, carrot extract, iris root extract, white birch extract, *Salvia miltorrhiza* extract, and Laminaria algae extract. The neem seed cell broth is comprised of tissue cultured neem seed cells and the respective cell culture medium. In this blend, it is contemplated that the neem seed cells can be separated from the broth and used absent the culture medium component or, alternatively, the culture medium component alone, which invariably contains seed cell constituents even after the seed cells have been separated therefrom, can be used instead.

A preferred blend has both neem seed cell broth and pomegranate fruit extract. A more preferred blend has neem seed cell broth and pomegranate fruit extract along with either or both of *Salvia miltiorrhiza* extract and grape seed extract.

The amount of the active botanical blend in the compositions of the present invention is from about 0.001 percentage by weight (wt%) to 50 wt% based on the total weight of the composition. Preferably, the amount is about 0.01 wt% to about 20 wt%. More preferably, the amount is about 0.1 wt% to about 10 wt%. Most preferably, the amount is about 3 wt% to about 8 wt%.

The blends of the present invention are preferably used in a cosmetically acceptable vehicle. Examples of cosmetically acceptable vehicles suitable for all embodiments of the present invention include, but are not limited to, water, glycerin, various alcohols such as ethanol, propyl alcohol, vegetable oil, mineral oil, silicone oils, fatty ethers, fatty esters, fatty alcohols, glycols, polyglycols or any combinations thereof.

Neem seed cell broth is prepared by tissue culture of cells isolated from neem seed. Neem seed cells are cultured in tissue culture medium containing appropriate nutrients and ingredients. This process provides for rapid cell growth and the production of key active compounds from the neem seed cell broth. The use of cell bioengineering in the form of plant tissue culture provides a standardized predictably high potency supply of this ingredient in the botanical blend of this invention. The Inventors' *in vitro* studies have shown that neem seed cell broth improves cell proliferation of keratinocytes, increases fibroblast metabolism, decreases skin pigmentation, decreases stress induced aging effects and increases pro-collagen synthesis. Additional teachings to neem seed cell extract and neem seed cell broth are disclosed in copending application entitled "Topical Compositions Having Undifferentiated Plant Seed Cells and Method for Using Same," filed November 9, 2001, the entire disclosure of which is incorporated herein by reference.

In cosmetic compositions of the present invention, the preferred amount of neem seed cell broth is about 0.001 wt% to about 50 wt%, preferably amount about 0.01 wt% to about 10 wt%, and more preferably about 0.1 wt% to about 5.0 wt%, based on the total weight of the composition.

The pomegranate fruit extract is an aqueous extract of the whole fruit, including the seed, peel, pulp, and juice. The extract is preferably non-carbonated. The Inventors' *in vitro* studies have shown that pomegranate fruit extract decreases pigmentation, binds effectively to estrogen receptor, inhibits the activity of elastase and collagenase, and increases pro-collagen synthesis. When used in the cosmetic compositions of the present invention, the preferred amount of pomegranate fruit extract is about 0.01 wt% to about 10.0 wt% and preferably about 0.1 wt% to about 5.0 wt% based on the total weight of the composition.

Danshen, or extract of *Salvia miltiorrhiza,* is useful in the present invention. The Inventors' *in vitro* studies have shown that *Salvia miltiorrhiza* extract increases fibroblast metabolism and increased pro-collagen synthesis. When used in the present cosmetic compositions, the preferred amount of *Salvia miltiorrhiza* extract is about 0.01 wt% to about 10 wt% and preferably about 0.1 wt% to about 5.0 wt% based on the total weight of the composition.

Grape seed extract is useful in the present invention. The Inventors' *in vitro* studies have shown that grape seed extract increases fibroblast metabolism, decreases pigmentation, and binds effectively to estrogen receptor. When used in the present cosmetic compositions, the preferred amount of grape seed extract is about 0.01 wt% to about 10 wt%, and preferably about 0.1 wt% to about 5.0 wt%, based on the total weight of the composition.

Cucumber extract is useful in the present invention. The Inventors' *in vitro* studies have shown that cucumber extract increases fibroblast metabolism, decreases pigmentation, and binds effectively to estrogen receptor. When used in the present cosmetic compositions, the preferred amount of cucumber extract is about 0.01 wt% to about 10 wt%, and preferably about 0.1 wt% to about 5.0 wt%, based on the total weight of the composition.

Carrot extract is useful in the present invention. Carrot extract contains b-carotene and vitamin A. The Inventors' *in vitro* studies have shown that carrot extract decreases skin pigmentation, binds effectively to estrogen receptor, and increases pro-collagen synthesis. When used in the present cosmetic compositions, the preferred amount of carrot extract is about 0.01 wt% to about 10 wt%, and preferably about 0.01 wt% to about 5.0 wt%, based on the total weight of the composition.

Iris root extract, an extract of the root of *Iris florentina,* is useful in the present invention. The Inventors' *in vitro* studies have shown that iris root extract increases fibroblast metabolism and decreases pigmentation. Preferably, the iris root extract useful in the present invention has from about 1.2 grams/liter to about 2.2 grams/liter of genistein equivalent isoflavones. When used in the present cosmetic compositions, the preferred amount of iris root extract is about 0.1 wt% to about 10 wt%, and preferably about 0.1 wt% to about 5.0 wt%, based on the total weight of the composition.

White birch extract is useful in the present invention. The Inventors' *in vitro* studies have shown that white birch extract increases fibroblast metabolism and decreases pigmentation. When used in the present cosmetic compositions, the preferred amount of white birch extract is about 0.001 wt% to about 10 wt%, and preferably about 0.01 wt% to about 5.0 wt%, based on the total weight of the composition.

Laminaria algae extract is useful in the present invention. The Inventors' *in vitro* studies have shown that Laminaria algae extract increases fibroblast metabolism and decreases pigmentation. When used in the present cosmetic compositions, the preferred amount of Laminaria algae extract is about 0.001 wt% to about 10 wt%, and preferably about 0.01 wt% to about 2.0 wt%, based on the total weight of the composition.

The compositions of the present invention may be formulated in any convenient form suitable for topical application to the skin. Such forms include aerosol spray, gel, cream, dispersion, emulsion, foam, liquid, lotion, mousse, patch, pomade, powder, pump spray, solid, solution, stick or towelette. The preferred cosmetic form is a cream that is an oil-in-water emulsion. Water-in-oil and water-in-silicone emulsions are also comtemplated.

The present invention is further illustrated by the following examples, which are intended only for illustration of the present invention and not for limitation of the scope thereof.

### Example 1

| Oil-in-Water. Emulsion | |
|---|---|
| Humectant (e.g. glycols, glycerols) | 0.5-15% |
| Thickeners (e.g. gums, starches, polymers) | 0.14% |
| Chelants I(e.g, disodium edta. tetrasodium EDTA) | 0.001-0.5% |
| Preservatives | 0.01-2% |
| Sunscreen (e.g. benzophenone, ethylhexylmetnoxycinnamate) | 0.1-050% |
| Silicone | 0.1-15% |
| Silica | 0.01-10% |
| Fatty Alcohol/ Emulsifers /Wax/ Fatty Acid | 0.5-15% |
| Emollients | 0.1-20% |
| Extracts neem seed cell broth (e.g. one or more of pomegranate extract, | |
| grape seed extract, | |
| salvia miltiorrhiza extract, iris florentina root extract, | |
| carrot extract, cucumber extract, | |
| white birch(betula alba) bark extract, | |
| rosemary extract, algae extract, or any combination) | 0.0001-50% |
| Demineralized Water | Q.S. |

### Example 2

| Oil-In-Water Emulsion | |
|---|---|
| Humectant (e,g, Glycols, Glycerols) | 0.5-15% |
| Thickeners (e.g. Gums, Starches, Polymers) | 0.1-4% |
| Chelants I(e.g. Disodium EDTA. Tetrasodium EDTA) | 0.001-0.5% |
| Preservatives | 0.01-2% |
| Sunscreen (e.g, Parsol 1789, ethylhexylmethoxycinnamate, | |
| benzophenone-3) | 0,1-50% |
| Silicone | 0.1-15% |
| Silica | 0.01-10% |
| Fatty alcohol/ Emulslfers /Wax/ Fatty acid (e.g. ceteth-20 | |
| phosphate/cetearyl alcohol/dicetyl phosphate, Tribehenh PEG-20 Ester, Sodium Dihydroxycethyl phosphate, cetearyl glocoside, cocoglyceride, | |
| | 0.5-15% |
| Emulsion Stabilizers/Viscosity Modifiers(e.g. acrylates/C₁₀₋₃₀ Alkyl acrylate Crosspolymer, Acrylate/Aminoacrylates/C₁₀₋₃₀ Alkyl PEG-20 Itaconate, Sodium Acrylate/Acryloyldimethyl Taurate Coploymer, Acrylates/C₁₀₋₃₀ Alkyl Acrylate Crosspolymer | 0.1-20% |
| Film Formers (e.g. decene/butene copolymer, acrylates/octylacrylamide copolymer, adipic acid/diethylene glycol/glycerin crosspolymer | 0.001-2% |
| Emollients | 0.1-20% |
| Pomegranate extract | 0.01-2% |
| Neem seed cell broth | 0.01-10% |
| Grape seed extract | 0.01-2% |
| Salvia Miltiorrhiza extract | 0.01-10% |
| Iris florentina root extract | 0.00-20% |
| Carrot extract | 0.00-2% |
| Cucumber extract | 0.00-2% |
| White birch(Betula Alba) Bark extract | 0.00-2% |
| Rosemary extract | 0.00-2% |
| Algae extract | 0.00-2% |
| Demineralized Water | Q.S. |

### Example 3

| Water/Silicone Emulsion | |
|---|---|
| Sodium PCA 50% | 0.1-4% |
| Sodium Lactate 60% | 0.01-10% |
| Sodium Chloride | 0.1-10% |
| Humectant(Glycerin, Glycols, Glycerols) | 0.5-10%, |
| Ammonium Hydroxide | 0.01-10% |
| Cyclomethicone | 0.1-20% |
| Cyclomethicone/Dimethicone Copolyol | 0.1-20% |
| Emollients(E.G. Cetyl Octanoate) | 0.1-20% |
| Dimethicone Copolyol/Cyclopentasiloxane | 0.1-10% |
| Pomegranate Extract | 0.01-2% |
| Neem Seed Cell Broth | 0.01-10% |
| Grape Seed Extract | 0.00-2% |
| Salvia Miltiorrhiza Extract | 0.00-10% |
| Iris Florentina Root Extract | 0.00-20% |
| Carrot Extract | 0.00-2% |
| Cucumber Extract | 0.00-2% |
| White Birch(Betula Alba) Bark Extract | 0.00-2% |
| Rosemary Extract | 0.00-2% |
| Algae Extract | 0.00-2% |
| Demineralized Water | Q.S |

### Example 4

| Gel | |
|---|---|
| Carbopol | 0.01-3% |
| Glycerin | 0.1-30% |
| Butylene Glycol | 0.1-30% |
| Disodium Edta | 0.01-2% |
| Methylparaben | 0.01-2% |
| Hydroxyethyl Cellulose | 0.01-2% |
| Corn (Zea Mays) Starch | 0.01-10% |
| C.S. D&C Yellow No.10 | 0.001-1% |
| C.S. Fd&C Blue No. 1 | 0.001-1 % |
| POE (20M) METHYL GLUCOSE ETHER | 0.01-10% |
| DIMETHYL POLYSILOXANE | 0.01-10% |
| PEG 50 SHEA BUTTER | 0.01-10% |
| SODIUM HYDROXIDE SOLUTION | 0.01-5% |
| BENZYL ALCOHOL | 0.01-5% |
| POMEGRANATE EXTRACT | 0.01-2% |
| NEEM SEED CELL BROTH | 0.00-10% |
| GRAPE SEED EXTRACT | 0.00-2% |
| Salvia Miltiorrhiza Extract | 0.00-10% |
| Iris Florentina Root Extract | 0.00-20% |
| Carrot Extract | 0.00-2% |
| Cucumber Extract | 0.00-2% |
| White Birch(Betula Alba) Bark Extract | 0.00-2% |
| Rosemary Extract | 0.00-2% |
| Algae Extract | 0.00-2% |
| Demineralized Water | Q.S |

### Example 5

| Cleansing Foam | |
|---|---|
| Humectant (Glycerin, Butylene Glycol) | 5-25% |
| Polyethylene Glycol | 0.1-20% |
| Bentonite | 0.1-20% |
| Stearic Acid | 0.1-30% |
| Myristic Acid | 0.1-20% |
| Cetearyl Alcohol/Ceteareth-20 | 1.00000% |
| Potassium Hydroxide 45% | 0.1-20% |
| Preservatives(E.G. Benzyl Alcohol, | 0.1-10% |
| 2-Phenoxyethanol, Benzyl Alcohol) | |
| Pomegranate Extract | 0.00-2% |
| Neem Seed Cell Broth | 0.01-10% |
| Grape Seed Extract | 0.01-2% |
| Salvia Miltiorrhiza Extract | 0.00-10% |
| Iris Florentina Root Extract | 0.00-20% |
| Carrot Extract | 0.00-2% |
| Cucumber Extract | 0.00-2% |
| White Birch(Betula Alba) Bark Extract | 0.00-2% |
| Rosemary Extract | 0.00-2% |
| Algae Extract | 0.00-2% |
| Demineralized Water | Q.S. |

Although the present invention describes in detail certain embodiments, it is understood that variations and modifications exist known to those skilled in the art that are within the invention. Accordingly, the present invention is intended to encompass all such alternatives, modifications and variations that are within the scope of the invention as set forth in the following claims.

## Claims

1. A topical cosmetic composition for improving the aesthetic appearance of skin comprising:
a blend of neem seed cell broth and one or more botanical ingredients selected from the group consisting of *Salvia miltorrhiza* extract, pomegranate fruit extract, grape seed extract, cucumber extract, carrot extract, Iris root extract, white birch extract, and Laminaria algae extract.

2. The composition of claim 1, further comprising a cosmetically acceptable vehicle.

3. The composition of claim 1, wherein the blend is in an amount from about 0.001 wt% to about 50 wt% based on the total weight of the composition,

4. The composition of claim 1, wherein the blend has *Salvia miltorrhiza* extract,

5. The composition of claim 4, wherein the neem seed cell broth is present in an amount from about 0.001 wt% to about 50 wt%, and wherein the *Salvia miltorrhiza* extract is present in an amount from about 0,1 wt% to about 10 wt%, based on the total weight of the composition.

6. The composition of claim 1, wherein the blend has pomegranate fruit extract.

7. The composition of claim 6, wherein the neem seed cell broth is present In an amount from about 0.001 wt% to about 50 wt%, and wherein the pomegranate fruit extract is present in an amount from about 0.01 wt% to about 10 wt%, based on the total weight of the composition.

8. The composition of claim 1, wherein the blend has *Salvia miltorrhiza* extract and pomegranate fruit extract.

9. The composition of claim 8, wherein the neem seed cell broth is present in an amount from about 0,001 wt% to about 50 wt%, wherein the *Salvia miltorrhi*za extract is present in an amount from about 0.1 wt% to about 10 wt%, and wherein the pomegranate fruit extract is present in an amount from about 0.01 wt% to about 10.0 wt%, based on the total weight of the composition,

10. The composition of claim 1, wherein the blend has *Salvia miltorrhiza* extract, pomegranate fruit extract, and grape seed extract.

11. A cosmetic method for improving the aesthetic appearance of skin comprising topically applying to the skin a cosmetically effective amount of the composition according to claim 1.

12. The cosmetic method of claim 11, wherein the improvements, are one or more improvements selected from the group consisting of: reduction in dermtological signs of chronological aging, hormonal aging and/or photoaging; reduction In skin fragility; reduction in pore size; prevention and/or reversal of loss of collagen and/or elastin; prevention of skin atrophy; prevention and/or reduction in appearance and/or depth of lines and/or wrinkles; prevention reduction and/or treatment of hyperpigmentation; improvement in skin tone, radiance, clarity and/or tautness; prevention, reduction, and/or amelioration of skin sagging; promotion of anti-oxidant activity; improvement in skin firmness, plumpness, suppleness and/or softness; improvement in procollagen and/or collagen production; improvement in skin texture and/or promotion of retexturization; improvement in skin barrier repair and/or function; improvement in appearance of skin contours; restoration of skin luster and/or brightness; minimization of dermatological signs of fatigue and/or stress; resistance to environmental stress; replenishment of essential nutrient and/or constituents of in the skin decreased by aging and/or menopause; increase in cell proliferation and/or multiplication; retardation of cellular aging; inhibition of enzymes in the skin that accelerate aging of skin calls; minimization of skin dryness and/or improvement in skin moisturization; minimization of skin discoloration, promotion and/or acceleration of cell turnover; enhancement of skin thickness; increase in skin elasticity and/or resiliency; and enhancement of exfoliation.

13. The cosmetic method of claim 11, wherein the improvements are one or more improvements selected from the group consisting of; reduction in dermatological signs of chronological aging, hormonal aging and photo aging; prevention and/or reduction in appearance and/or depth of lines and/or Wrinkles; improvement in skin tone, radiance, clarity and/of tautness; improvement In skin firmness, plumpness, suppleness, and/or softness; and improvement in skin texture and/or promotion of retexturization.

14. The cosmetic method of claim 11, wherein the improvements are one or more improvements selected from the group consisting of minimization of dermatological signs of fatigue and/or stress; resistance to environmental stress; Improvement in procollagen and/or collagen production; prevention and/or reversal of loss of collagen and/or elastin; minimization of skin dryness and/or improvement in skin moisturization: enhancement of skin thickness; increase in skin elasticity and/or resiliency; and enhancement of exfoliation,

15. A cosmetic method of treating skin comprising topically applying to the skin the composition of claim 1 In an amount effective to prevent, ameliorate, inhibit and/or reduce signs of dermatological aging.

16. The cosmetic method of claim 15, wherein the signs of aging are skin fragility: loss of collagen and/or elastin; estrogen imbalance in skin; skin atrophy; appearance and/or depth of lines and wrinkles; skin discoloration; skin sagging: skin fatigue and/or stress; skin dryness; skin flakiness; cellular aging; loss of skin tone, clarity and/or luster; loss of skin firmness; poor skin texture; loss of skin elasticity and/or resiliency; and thin skin.

## Patentansprüche

1. Topische Kosmetik-Zusammensetzung zur Verbesserung des ästhetischen Aussehens der Haut, umfassend:
ein Gemisch aus Niemkernzellbrühe und einem oder mehreren pflanzlichen Inhaltsstoffen, ausgewählt aus der Gruppe, bestehend aus *Salvia miltorrhiza-Extrakt,* Granatapfel-Fruchtextrakt, Traubenkernextrakt, Gurkenextrakt, Karottenextrakt, Iriswurzelextrakt, Weißbirkenextrakt, und Laminaria-Algenextrakt,

2. Zusammensetzung nach Anspruch 1, zudem umfassend eine kosmetisch verträgliche Trägersubstanz.

3. Zusammensetzung nach Anspruch 1, wobei das Gemisch in einer Menge von etwa 0,001 Gew.-% bis etwa 50 Gew.-%, bezogen auf das Gesamtgewicht der Zusammensetzung, vorliegt.

4. Zusammensetzung nach Anspruch 1, wobei das Gemisch den *Salvia miltorrhiza-Extrakt* enthält.

5. Zusammensetzung nach Anspruch 4, wobei die Niemkernzellbrühe in einer Menge von etwa 0,001 Gew.-% bis etwa 50 Gew.-% vorliegt, und wobei der *Salvia miltorrhiza*-Extrakt in einer Menge von etwa 0,1 Gew.-% bis etwa 10 Gew,-%, bezogen auf das Gesamtgewicht der Zusammensetzung, vorliegt.

6. Zusammensetzung nach Anspruch 1, wobei das Gemisch den Granatapfel-Fruchtextrakt enthält.

7. Zusammensetzung nach Anspruch 6, wobei die Niemkernzellbrühe in einer Menge von etwa 0,001 Gew.-% bis etwa 50 Gew.-% vorliegt, und wobei der Granatapfel-Fruchtextrakt in einer Menge von etwa 0,01 Gew.% bis etwa 10 Gew,%, bezogen auf das Gesamtgewicht der Zusammensetzung vorliegt.

8. Zusammensetzung nach Anspruch 1, wobei das Gemisch den *Salvia miltorrhiza-Extrakt* und den Granatapfel-Fruchtapfelextrakt enthält.

9. Zusammensetzung nach Anspruch 8, wobei die Niemkernzellbrühe in einer Menge von etwa 0,001 Gew.-% bis etwa 50 Gew.-% vorliegt, wobei der *Salvia miltorrhiza*-Extrakt in einer Menge von etwa 0,1 Gew.-% bis etwa 10 Gew.-% vorliegt, und wobei der Granatapfel-Fruchtextrakt in einer Menge von etwa 0,01 Gew.-% bis etwa 10,0 Gew.-%, bezogen auf das Gesamtgewicht der Zusammensetzung vorliegt.

10. Zusammensetzung nach Anspruch 1, wobei das Gemisch den *Salvia miltorrhiza*-Extrakt, den Granatapfel-Fruchtextrakt und den Traubenkernextrakt enthält.

11. Kosmetisches Verfahren zur Verbesserung des ästhetischen Aussehens der Haut, umfassend das topische Auftragen einer kosmetisch wirksamen Menge der Zusammensetzung nach Anspruch 1 auf die Haut.

12. Kosmetisches Verfahren nach Anspruch 11, wobei es sich bei den Verbesserungen um eine oder mehrere Verbesserungen handelt, ausgewählt aus der Gruppe, bestehend aus: Reduktion der dermatologischen Anzeichen der chronologischen Alterung, hormonellen Alterung und/oder Lichtalterung; Reduktion der Hautbrüchigkeit; Reduktion der Porengröße; Verhinderung und/oder Umkehr des Verlusts von Kollagen und/oder Elastin; Verhinderung der Hautatrophie; Verhinderung und/oder Reduktion des Auftretens und/oder der Tiefe von Falten und/oder Runzeln; Verhinderung, Reduktion und/oder Behandlung von Hyperpigmentierung; Verbesserung von Hautfarbton, Glanz, Klarheit und/oder Straffheit; Verhinderung, Reduktion und/oder Verbesserung von Hautschlaffheit; Förderung der antioxidativen Aktivität, Verbesserung der Hautfestigkeit, Rundlichkeit, Geschmeidigkeit und/oder Weichheit; Verbesserung der Prokollagen- und/oder Kollagenproduktion; Verbesserung der Hautstruktur und/oder Förderung der Restrukturierung; Verbesserung der Reparatur und/oder Funktion der Hautbarriere; Verbesserung des Aussehens der Hautkonturen; Wiederherstellung von Hautglanz und/oder Helligkeit; Minimierung dermatologischer Anzeichen von Ermüdung und/oder Stress; Beständigkeit gegenüber Umweltbelastung; Ergänzung essentieller Nährstoffe und/oder Bestandteile davon in der Haut, welche durch Alterung und/oder Menopause verringert wurden; Steigerung der Zellproliferation und/oder-Vermehrung; Verzögerung der Zellalterung; Hemmung von Enzymen in der Haut, die die Alterung von Hautzellen beschleunigen; Minimierung der Hauttrockenheit und/oder Verbesserung der Hautbefeuchtung; Minimierung der Hautverfärbung; Förderung und/oder Beschleunigung des Zellumsatzes; Steigerung der Hautdicke; Steigerung der Hautelastizität und/oder -Spannkraft; und Steigerung der Exfoliation.

13. Kosmetisches Verfahren nach Anspruch 11, wobei es sich bei den Verbesserungen um eine oder mehrere Verbesserungen handelt, ausgewählt aus der Gruppe, bestehend aus: Reduktion der dermatologischen Anzeichen der chronologischen Alterung, hormonellen Alterung und Lichtalterung; Verhinderung und/oder Reduktion des Auftretens und/oder der Tiefe von Falten und/oder Runzeln; Verbesserung von Hautfarbton, Glanz, Klarheit und/oder Straffheit; Verbesserung der Hautfestigkeit, Rundlichkeit, Geschmeidigkeit und/oder Weichheit; und Verbesserung der Hautstruktur und/oder Förderung der Restrukturierung.

14. Kosmetisches Verfahren nach Anspruch 11, wobei es sich bei den Verbesserungen um eine oder mehrere Verbesserungen handelt, ausgewählt aus der Gruppe, bestehend aus, Minimierung dermatologischer Anzeichen von Ermüdung und/oder Stress; Beständigkeit gegenüber Umweltbelastung; Verbesserung der Prokollagen- und/oder Kollagenproduktion; Verhinderung und/oder Umkehr des Verlusts von Kollagen und/oder Elastin; Minimierung der Hauttrockenheit und/oder Verbesserung der Hautbefeuchtung; Steigerung der Hautdicke; Steigerung der Hautelastizität und/oder -Spannkraft und Steigerung der Exfoliation.

15. Kosmetisches Verfahren zur Behandlung von Haut, umfassend das topische Auftragen der Zusammensetzung nach Anspruch 1 auf die Haut in einer Menge, die Anzeichen dermatologischer Alterung effizient verhindert, verbessert, hemmt und/oder reduziert.

16. Kosmetisches Verfahren nach Anspruch 15, wobei es sich bei den Anzeichen der Hautalterung um Hautbrüchlgkelt; Verlust von Kollagen und/oder Elastin; Östrogen-Ungleichgewicht in der Haut; Hautatrophie; Auftreten und/oder Tiefe von Falten und Runzeln; Hautverfärbung; Hautschlaffheit, Hautermüdung und/oder Stress; Hauttrockenheit; Hautschuppigkeit; Zellalterung; Verlust des Hautfarbton; Klarheit und/oder Glanz; Verlust der Hautfestigkeit; schlechte Hautstruktur; Verlust der Hautelastizität und/oder -Spannkraft; und dünne Haut handelt.

## Revendications

1. Composition cosmétique topique pour l'amélioration de l'apparence esthétique de la peau qui comprend :
un mélange d'un bouillon de cellules de graines de margousier et d'un ou plusieurs ingrédients végétaux choisis parmi le groupe constitué d'extraits de *Salvia miltorrhiza,* de grenade, de pépins de raisin, de concombre, de carotte, de racine d'Iris, de bouleau blanc et d'algues laminaires.

2. Composition selon la revendication 1, qui comprend en outre un excipient acceptable sur le plan cosmétique.

3. Composition selon la revendication 1, dans laquelle le mélange est présent en une quantité d'environ 0,001 % en poids à environ 50 % en poids basée sur le poids total de la composition.

4. Composition selon la revendication 1, dans laquelle un extrait de *Salvia miltorrhiza* est présent dans le mélange.

5. Composition selon la revendication 4, dans laquelle le bouillon de cellules de graines de margousier est présent en une quantité d'environ 0,001 % en poids à environ 50 % en poids et dans laquelle l'extrait de *Salvia milthorrhiza* est présent en une quantité d'environ 0,1 % en poids à environ 10 % en poids, quantités basées sur le poids total de la composition.

6. Composition selon la revendication 1, dans laquelle un extrait de grenade est présent dans le mélange.

7. Composition selon la revendication 6, dans laquelle le bouillon de cellules de graines de margousier est présent en une quantité d'environ 0,001 % en poids à environ 50% en poids et dans laquelle l'extrait de grenade est présent en une quantité d'environ 0,01% en poids à environ 10 % en poids, quantités basées sur le poids total de la composition.

8. Composition selon la revendication 1, dans laquelle un extrait de *Salvia miltorrhiza* et un extrait de grenade sont présents dans le mélange.

9. Composition selon la revendication 8, dans laquelle le bouillon de cellules de graines de margousier est présent en une quantité d'environ 0,001 % en poids à environ 50 % en poids, dans laquelle l'extrait de *Salvia milthorrhiza* est présent en une quantité d'environ 0,1 % en poids à environ 10 % en poids et dans laquelle l'extrait de grenade est présent en une quantité d'environ 0,01 % en poids à environ 10 % en poids, quantités basées sur le poids total de la composition.

10. Composition selon la revendication 1, dans laquelle un extrait de *Salvia miltorrhiza,* un extrait de grenade et un extrait de pépins de raisin sont présents dans le mélange.

11. Procédé cosmétique d'amélioration de l'apparence esthétique de la peau comprenant l'application topique sur la peau d'une quantité efficace sur le plan cosmétique de la composition selon la revendication 1,

12. Procédé cosmétique selon la revendication 11, dans lequel les améliorations sont une ou plusieurs améliorations choisies parmi le groupe constitué : de la réduction des signes dermatologiques de vieillissement chronologique, de vieillissement hormonal et/ou de photovieillissement ; de la réduction de la fragilité de la peau ; de la réduction de la dimension des pores ; de la prévention et/ou de l'inversion de la perte de collagène et/ou d'élastine; de la prévention de l'atrophie de la peau; de la prévention et/ou de la réduction en apparence et/ou en profondeur des ridules et/ou de rides ; de la prévention, de la réduction et/ou du traitement de l'hyperpigmentation ; de l'amélioration du teint, de l'éclat, de la clarté et/ou de la tension de la peau ; de la prévention, de la réduction et/ou de l'amélioration de l'affaissement de la peau ; de la promotion de l'activité antioxydante ; de l'amélioration de la fermeté, de la rondeur, de la souplesse et/ou de la douceur de la peau ; de l'amélioration de la production de procollagène et/ou de collagène ; de l'amélioration de la texture de la peau et/ou de la promotion de la retexturatlon ; de l'amélioration de la réparation et/ou de la fonction de la barrière cutanée ; de l'amélioration en apparence des contours de la peau ; de la restauration du lustre et/ou de la brillance de la peau ; de la minimisation des signes dermatologiques de fatigue et/ou de stress ; de la résistance au stress environnemental ; de la reconstitution des nutriments et/ou des constituants essentiels dans la peau qui sont réduits par le vieillissement et/ou la ménopause ; de l'augmentation de la prolifération et/ou de la multiplication cellulaire ; du ralentissement du vieillissement cellulaire ; de l'inhibition des enzymes cutanées qui accélèrent le vieillissement des cellules cutanées ; de la minimisation de la sécheresse de la peau et/ou de l'amélioration de l'hydratation de la peau ; de la minimisation de la décoloration cutanée ; de la promotion et/ou de l'accélération du renouvellement des cellules ; de l'amélioration de l'épaisseur de la peau ; de l'augmentation de l'élasticité et/ou de la résilience de la peau et de l'amélioration de l'exfoliation,

13. Procédé cosmétique selon la revendication 11, dans lequel les améliorations sont une ou plusieurs améliorations choisies parmi le groupe constitué : de la réduction des signes dermatologiques de vieillissement chronologique, de vieillissement hormonal et de photovieillissement ; de la prévention et/ou de la réduction de l'apparence et/ou de la profondeur des ridules et/ou des rides ; de l'amélioration du teint, de l'éclat, de la clarté et/ou de la tension de la peau ; de l'amélioration de la fermeté, de la rondeur, de la souplesse et/ou de la douceur de la peau et l'amélioration de la texture de la peau et/ou de la promotion de la retexturation.

14. Procédé cosmétique selon la revendication 11, dans lequel les améliorations sont une ou plusieurs améliorations choisies parmi le groupe constitué de la minimisation des signes dermatologiques de la fatigue et/ou du stress ; de la résistance au stress environnemental ; de l'amélioration de la production de procollagène et/ou de collagène ; de la prévention et/ou de l'inversion de la perte de collagène et/ou d'élastine ; de la minimisation de la sécheresse de la peau et/ou de l'amélioration de l'hydratation de la peau ; de l'amélioration de l'épaisseur de la peau ; de l'amélioration de l'élasticité et/ou de la résilience de la peau et de l'amélioration de l'exfoliation,

15. Procédé cosmétique pour le traitement de la peau comprenant l'application topique sur la peau de la composition selon la revendication 1 en une quantité efficace pour empêcher, améliorer, inhiber et/ou réduire les signes de vieillissement dermatologique.

16. Procédé cosmétique selon la revendication 15, dans lequel les signes de vieillissement sont la fragilité de la peau, la perte de collagène et/ou d'élastine, le déséquilibre d'oestrogène dans la peau, l'atrophie de la peau, l'apparence et/ou la profondeur des ridules et des rides, la décoloration de la peau, l'affaissement de la peau, la fatigue et/ou le stress de la peau, la sécheresse de la peau, le flaklness de la peau, le vieillissement cellulaire, la perte du teint, de la clarté et/ou du lustre de la peau, la perte de la fermeté de la peau, la pauvre texture de la peau, la perte de l'élasticité et/ou de la résilience de la peau et la minceur de la peau.
